# EUROPEAN PATENT APPLICATION

(11) **EP 4 160 207 A1**
(43) Date of publication of application: **05.04.2023**
(21) Application number: 21818801.9
(22) Date of filing: 02.06.2021
(51) Int. Cl.: G01N 33/15, G01N 33/50, G01N 33/543

(54) **COMPOUND FIXING METHOD, DETECTION METHOD, SCREENING METHOD, SUBSTRATE USED IN SAME, COMPOUND FIXING AGENT, AND FIXING KIT**

(30) Priority: 02.06.2020 JP 2020095953
(71) Applicant: Public University Corporation Fukushima Medical University, Fukushima City Fukushima, 960-1295 (JP); Medicrome, Inc., Fukushima-shi, Fukushima 960-8031 (JP)
(72) Inventor: WATANABE Shinya, Fukushima City Fukushima 9601295 (JP); IMAI Junichi, Fukushima City Fukushima 9601295 (JP); MATSUKURA Susumu, Yokohama City Kanagawa 230-0046 (JP)
(74) Representative: Betten & Resch
(86) International application number: PCT/JP2021/021066
(87) International publication number: WO 2021/246456

(57) **Abstract**

To provide a method of easily immobilizing a compound with a wide variety of structures and functional groups onto a substrate without requiring modification and while maintaining the structures as is, a method of detecting this immobilized compound, and a compound screening method, and a screening substrate, a compound immobilizing agent, and an immobilizing kit used therein.

The method of immobilizing a compound onto a substrate includes immobilizing the compound as a protein-compound complex onto the substrate via a protein for immobilizing the compound onto the substrate. Further, the compound screening method includes detecting a compound having affinity for a target compound immobilized onto the substrate by bringing a candidate compound into contact with this target compound. A screening substrate on which the immobilizing protein forms a complex with a compound is also provided.

## Description

### Field of the Invention

The present invention relates to a method of immobilizing a compound onto a substrate, a method of detecting the compound, and a compound screening method, and a screening substrate, a compound immobilizing agent, and an immobilizing kit used therein.

### Description of the Background Art

In the field of chemical biology, examining what proteins bind to low-molecular compounds is very important research not only for basic research but also for the development of clinical drugs.

In general, a "compound microarray" is, as inspired by a DNA microarray, an organic compound immobilized onto a chip (or a glass slide) instead of DNA, which allows evaluation of physical interactions between target proteins and compounds. Although compound microarray technology is superior in principle as technology for efficient utilization of compound libraries, immobilizing compounds onto glass slides or special substrates has been difficult. Various studies have been conducted to overcome this challenge (for example,

### Non-Patent Document 1).

Methods of immobilizing compounds are broadly divided into methods of forming covalent bonds and methods of immobilizing by non-covalent bonds. Further, the methods of forming covalent bonds can be divided into methods that use and methods that do not use selective reactions between a specific functional group of the compound and a functional group introduced onto the substrate.

For example, among the immobilization methods of forming covalent bonds and attempting immobilization by using selective reactions, a method of immobilizing a thiol-containing compound library on a glass substrate with a maleimide group introduced thereon, a method of immobilizing an alcohol compound library by utilizing reactions with a silyl group or by using a diisocyanate group, a method of immobilizing a compound library having an acidic functional group by utilizing a diazobenzylidene group, a method of immobilizing an amine-containing compound library by utilizing reactions with an aldehyde group or by utilizing reactions with a trysil group, a method of immobilizing a cysteine-containing compound library by utilizing reactions with a glyoxyl group, a method of immobilizing a hydrazide-containing compound library by utilizing reactions with an epoxide group, a method of immobilizing an azide compound library by utilizing reactions with an alkyne, a method of immobilizing a cyclopentadiene-containing compound library by utilizing Diels-Alder reactions, and the like have been reported. Further, among the methods of attempting immobilization by using non-selective reactions, a method of immobilizing by utilizing reactions with an aryldiazirine group and the like have been reported.

Among the immobilization methods of forming non-covalent bonds and attempting immobilization by using selective reactions, a method of immobilizing by utilizing reactions between biotinylated compounds and avidin, a method of immobilizing compounds with a nucleic acid tag introduced therein, a method of immobilizing compounds with peptide nucleic acid introduced therein, and the like have been reported. Further, among the methods of attempting immobilization by using non-selective reactions, a method of causing the compound to be physically adsorbed in a nitrocellulose-coated substrate, a method of utilizing each spot of a compound solution as a reaction chamber, a method of using a substrate coated with a biodegradable polymer, and the like have been reported.

Nevertheless, these methods are based on the premise that each individual compound is separately immobilized, and cannot be used to immobilize an aggregate of compounds with a wide variety of structures and functional groups, as in compound libraries held by many research institutions, onto the same substrate. Further, each method also has disadvantages. For example, in a case in which a region on the compound that is used for immobilization onto the substrate overlaps a region that interacts with the protein, the immobilized compound is useless. Furthermore, a compound that has been structurally modified by introducing a tag or the like does not, in a strict sense, retain all of the properties of the original compound.

### PRIOR ART DOCUMENTSNon-Patent Documents[0008]

Non-Patent Document 1: Journal of Synthetic Organic Chemistry, Japan, Vol. 64, No. 6, 35-46 (2006)

### SUMMARY OF THE INVENTION

### Problems to be Solved by the Invention

An object of the present invention is to provide a method of easily immobilizing a compound with a wide variety of structures and functional groups onto a substrate without requiring modification and while maintaining the structures as is.

### Means for Solving the Problems

To solve the above-described problems, the inventors conducted diligent studies and found that a protein can readily form a complex simply by being mixed with a compound in a solvent, and that the complex can be immobilized onto a substrate via the protein. The present invention was completed on the basis of the above-described findings and includes the following aspects:
That is, as one aspect, the present invention relates to
[1] a compound immobilizing method of immobilizing a compound onto a substrate, the compound immobilizing method comprising
   immobilizing the compound as a protein-compound complex onto the substrate via an immobilizing protein for immobilizing the compound onto the substrate.

Here, the compound immobilizing method of the present invention, in one embodiment, is
[2] the compound immobilizing method described in [1] above, wherein
the substrate is glass.

Further, the compound immobilizing method of the present invention, in one embodiment, is
[3] the compound immobilizing method described in [1] or [2] above, wherein
the immobilizing protein is a blood protein or an analog thereof.

Further, the compound immobilizing method of the present invention, in one embodiment, is
[4] the compound immobilizing method described in any one of [1] to [3] above, wherein
the immobilization includes
(a) preparing a protein-compound complex by mixing the immobilizing protein and the compound in a solvent, and
(b) immobilizing the protein-compound complex onto the substrate.

Further, the compound immobilizing method of the present invention, in one embodiment, is
[5] the compound immobilizing method described in any one of [1] to [3] above, wherein
the immobilization includes
(a') immobilizing the immobilizing protein onto the substrate, and
(b')forming a protein-compound complex by bringing the compound into contact with the immobilizing protein immobilized onto the substrate.

Further, the compound immobilizing method of the present invention, in one embodiment, is
[6] the compound immobilizing method described in any one of [1] to [5] above, wherein
the protein-compound complex is formed by a non-covalent bond between the immobilizing protein and the compound.

Further, the compound immobilizing method of the present invention, in one embodiment, is
[7] the compound immobilizing method described in any one of [1] to [6] above, wherein
a plurality of the protein-compound complexes are immobilized onto the substrate, and the immobilizing protein and the compound are bonded to each other in different orientations across the plurality of protein-compound complexes.

Further, the compound immobilizing method of the present invention, in one embodiment, is
[8] the compound immobilizing method described in any one of [1] to [7] above, wherein
the compound forming the protein-compound complex maintains activity of the compound before formation of the protein-compound complex.

Further, the compound immobilizing method of the present invention, in one embodiment, is
[9] the compound immobilizing method described in any one of [1] to [8] above, wherein
the compound is a compound group including a plurality of compounds that differ from each other, and
the immobilization includes immobilizing the compound group onto the substrate via the immobilizing protein.

Further, the compound immobilizing method of the present invention, in one embodiment, is
[10] the compound immobilizing method described in [4] above, further comprising
(c) cleaning the substrate after step (b).

Further, the compound immobilizing method of the present invention, in one embodiment, is
[11] the compound immobilizing method described in [5] above, further comprising
(c') cleaning the substrate after step (a') or/and step (b').

Further, in another aspect, the present invention relates to
[12] a compound detecting method of detecting a compound immobilized onto a substrate, the compound detecting method comprising
detecting the compound immobilized onto the substrate by the compound immobilizing method described in any one of claims 1 to 11.

Here, the compound detecting method of the present invention, in one embodiment, is
[13] the compound detecting method described in [12] above, further comprising
recovering the protein-compound complex or the compound detected by the detection.

Further, in another aspect, the present invention relates to
[14] a compound screening method of screening a compound having affinity for a target compound, the compound screening method comprising
(i) immobilizing the target compound as a protein-compound complex onto a substrate via an immobilizing protein for immobilizing the compound onto the substrate, and
(ii) detecting the compound having affinity for the target compound immobilized onto the substrate by bringing a candidate compound into contact with the target compound.

Here, the compound screening method of the present invention, in one embodiment, is
[15] the compound screening method described in [14] above, wherein
the target compound is a compound having affinity for a disease-related marker, and the candidate compound is a compound derived from a biological sample, and
step (ii) includes detecting the disease-related marker in the biological sample by bringing the compound derived from the biological sample into contact with the compound having affinity for the disease-related marker and immobilized onto the substrate.

Further, the compound screening method of the present invention, in one embodiment, is
[16] the compound screening method described in [14] above, wherein
the candidate compound is a compound derived from a biological sample, and
a profile of a compound included in the biological sample with respect to the target compound is obtained.

Further, the compound screening method of the present invention, in one embodiment, is
[17] the compound screening method described in [16] above, wherein
the target compound is an antigen-candidate compound,
the candidate compound is an antibody included in a biological sample, and
a profile of an antibody included in the biological sample with respect to the antigen-candidate compound is obtained.

Further, in another aspect, the present invention relates to
[18] a compound immobilizing agent composed of an immobilizing protein for immobilizing a compound onto a substrate.

Further, in another aspect, the present invention relates to
[19] a screening substrate used in the compound detecting method described in [12] or [13] above or the compound screening method described in [14] or [15] above, wherein the immobilizing protein is bonded on a surface thereof.

Here, the substrate of the present invention, in one embodiment, is
[20] the screening substrate described in [19] above, wherein
the immobilizing protein forms a complex with a compound.

Further, in another aspect, the present invention relates to
[21] an immobilizing kit for immobilizing a compound onto a substrate, the immobilizing kit comprising
a substrate and an immobilizing protein.

### Effect of the Invention

According to the immobilization method of the present invention, it is possible to easily immobilize a compound with a wide variety of structures and functional groups onto a substrate without requiring separate modification and while maintaining the structures of the compound as is.

### BRIEF DESCRIPTION OF THE DRAWINGS

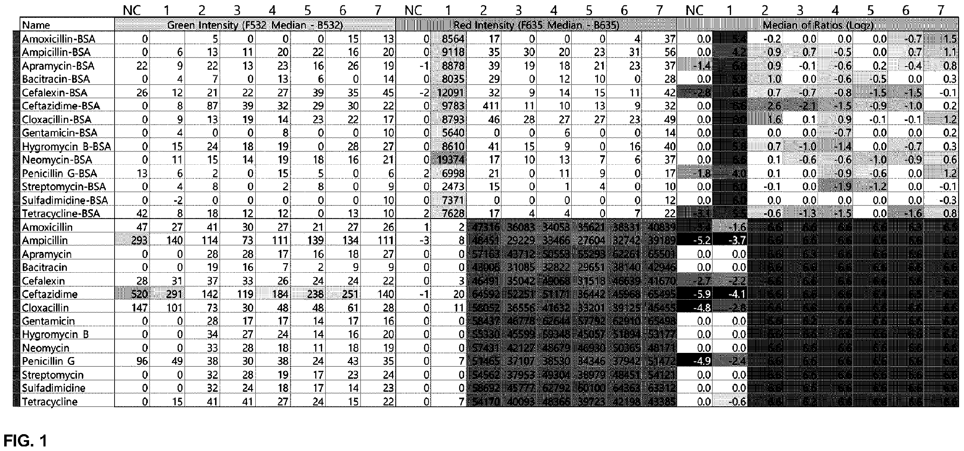

Fig. 1 is a table showing the results of immobilizing, onto substrates, complexes of 14 compounds (amoxicillin, ampicillin, apramycin, bacitracin, cefalexin, ceftazidime, cloxacillin, gentamicin, hygromycin B, neomycin, penicillin G, streptomycin, sulfadimidine, and tetracycline) and HSA, and compounds with the 14 compounds and BSA covalently bonded, and detecting HSA and BSA on the substrate by using six mouse monoclonal anti-HSA antibodies and one mouse monoclonal anti-BSA antibody as well as a fluorescence-labeled secondary antibody.

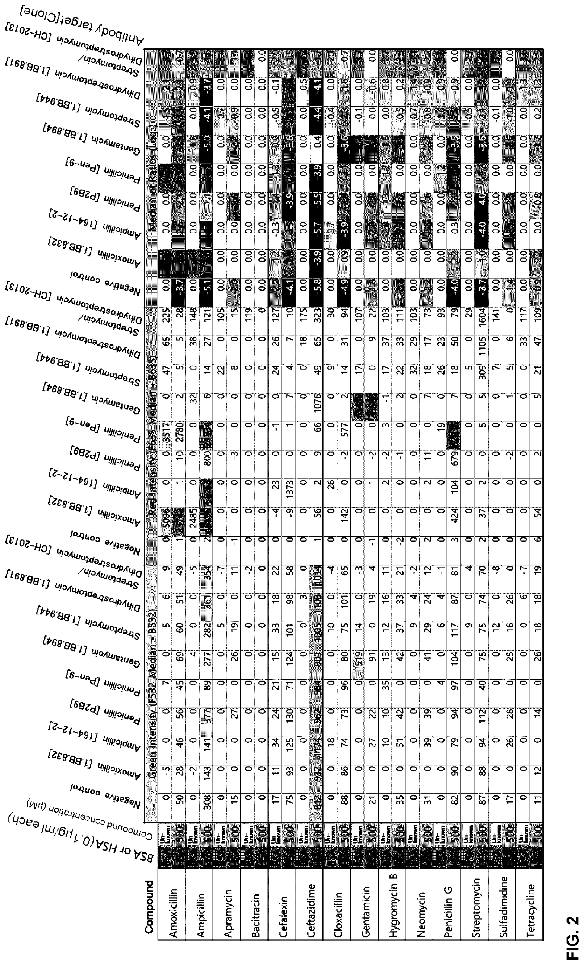

Fig. 2 is a table showing the results of immobilizing, onto substrates, complexes of 14 compounds (amoxicillin, ampicillin, apramycin, bacitracin, cefalexin, ceftazidime, cloxacillin, gentamicin, hygromycin B, neomycin, penicillin G, streptomycin, sulfadimidine, and tetracycline) and HSA, and compounds with the 14 compounds and BSA covalently bonded, and detecting each compound on the substrate by using an anti-amoxicillin antibody, an anti-ampicillin antibody, an anti-penicillin antibody, an anti-gentamycin antibody, an anti-dihydrostreptomycin antibody, and an anti-streptomycin/dihydrostreptomycin antibody as well as a fluorescence-labeled secondary antibody.

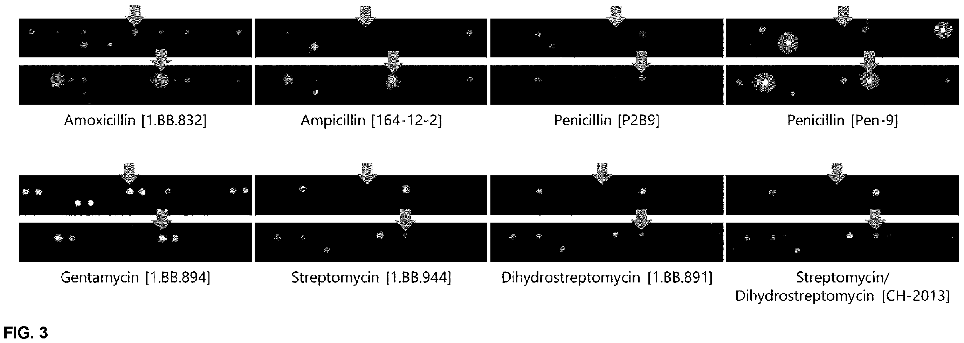

Fig. 3 shows images when compounds covalently bonded to BSA in advance or complexes of HSA and a compound, each immobilized onto a substrate, were detected by using a labeled antibody in Example 2 described below. For each compound, the upper image shows the detection result of the compound covalently bonded to BSA in advance, and the lower image shows the detection result of the complex of HSA and the compound. Each arrow indicates a position where the compound that is a target antigen of the antibody is spotted.

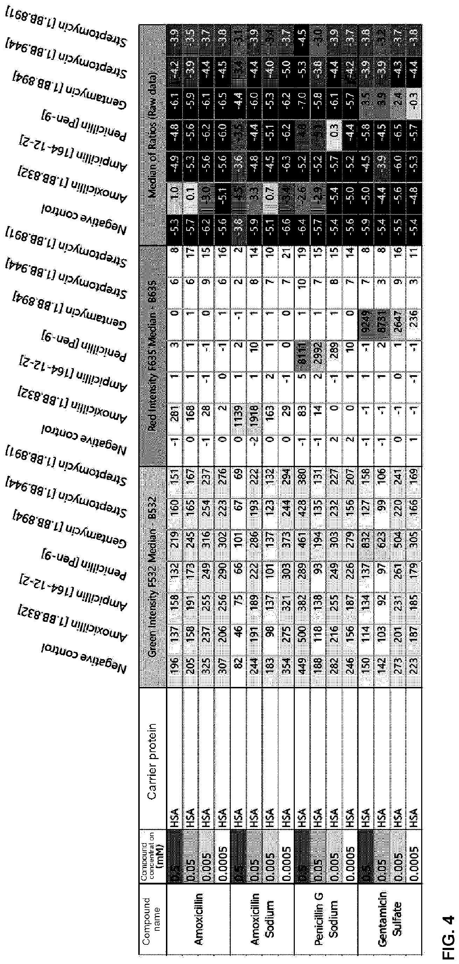

Fig. 4 is a table showing the results of detecting an effect of varying a concentration of HSA mixed with amoxicillin, amoxicillin sodium, penicillin G sodium, and gentamycin sulfate on immobilization onto the substrate by using labeled antibodies in Example 3 described below.

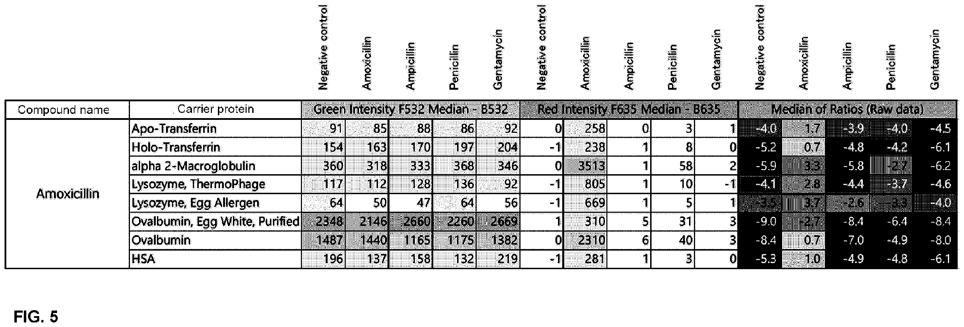

Fig. 5 is a table showing the results of immobilizing several complexes of an immobilizing protein and amoxicillin onto a substrate, and detecting each compound on the substrate by using an anti-amoxicillin antibody, an anti-ampicillin antibody, an anti-penicillin antibody, and an anti-gentamycin antibody as well as a fluorescence-labeled secondary antibody in Example 4 described below.

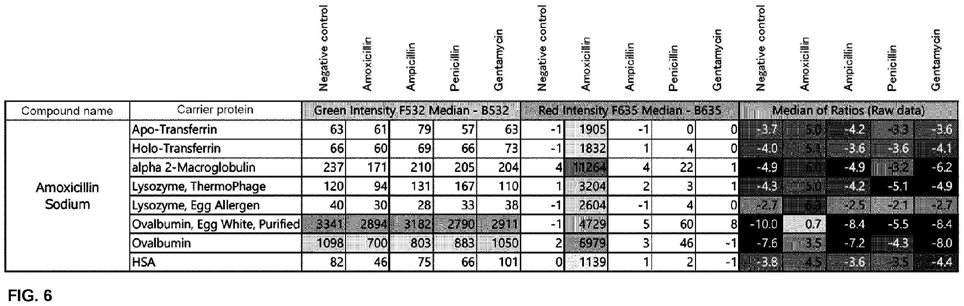

Fig. 6 shows the results of immobilizing complexes of an immobilizing protein and amoxicillin sodium onto a substrate, and detecting each compound on the substrate by using an anti-amoxicillin antibody, an anti-ampicillin antibody, an anti-penicillin antibody, and an anti-gentamycin antibody as well as a fluorescence-labeled secondary antibody in Example 4 described below.

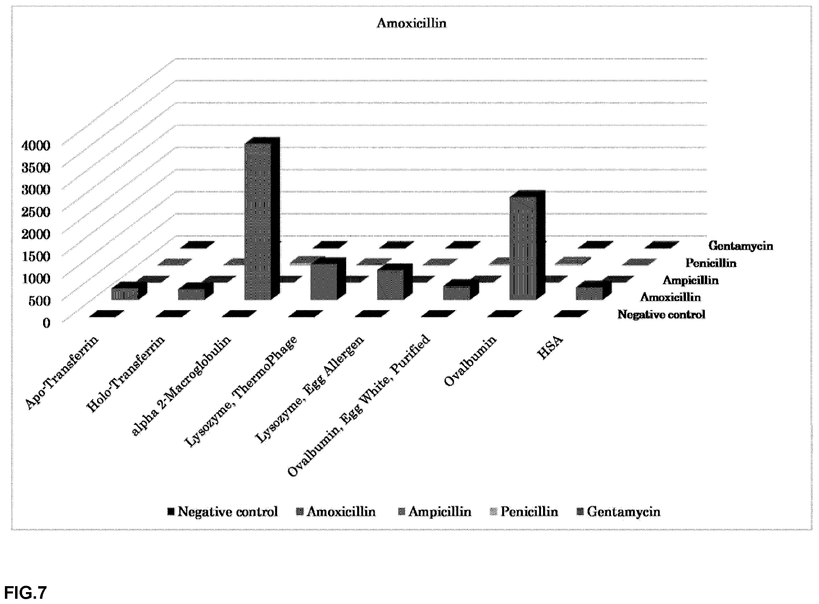

Fig. 7 is a graphical representation of the red intensity values in the table of Fig. 5.

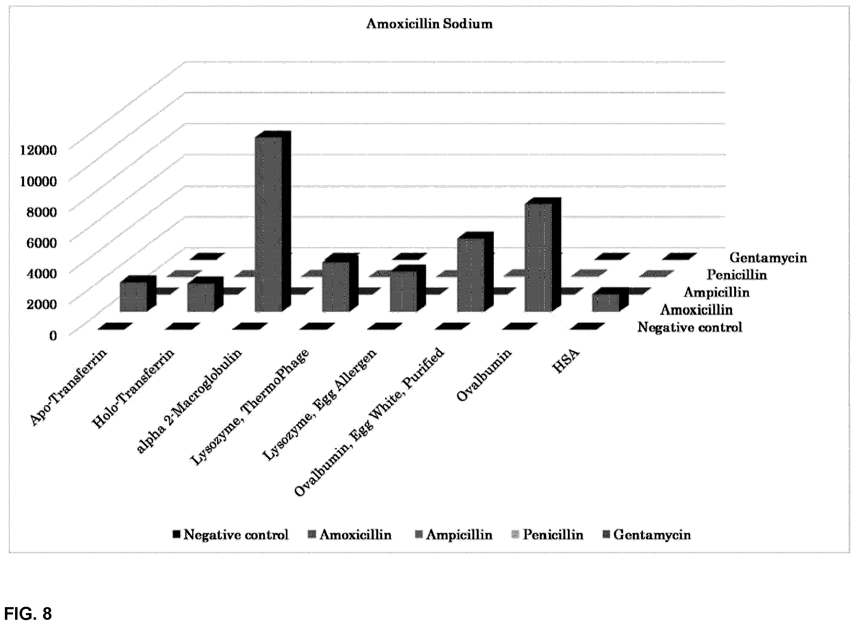

Fig. 8 is a graphical representation of the red intensity values in the table of Fig. 6.

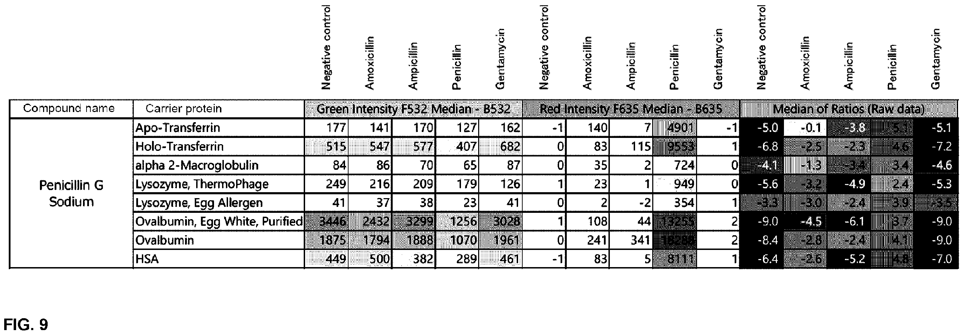

Fig. 9 is a table showing the results of immobilizing complexes of an immobilizing protein and penicillin G sodium onto a substrate, and detecting each compound on the substrate by using an anti-amoxicillin antibody, an anti-ampicillin antibody, an anti-penicillin antibody, and an anti-gentamycin antibody as well as a fluorescence-labeled secondary antibody in Example 4 described below.

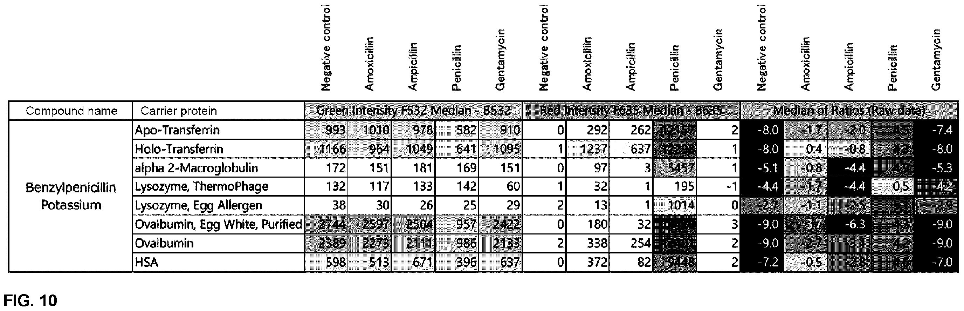

Fig. 10 is a table showing the results of immobilizing a complex of an immobilizing protein and benzyl penicillin potassium onto a substrate, and detecting each compound on the substrate by using an anti-amoxicillin antibody, an anti-ampicillin antibody, an anti-penicillin antibody, and an anti-gentamycin antibody as well as a fluorescence-labeled secondary antibody in Example 4 described below.

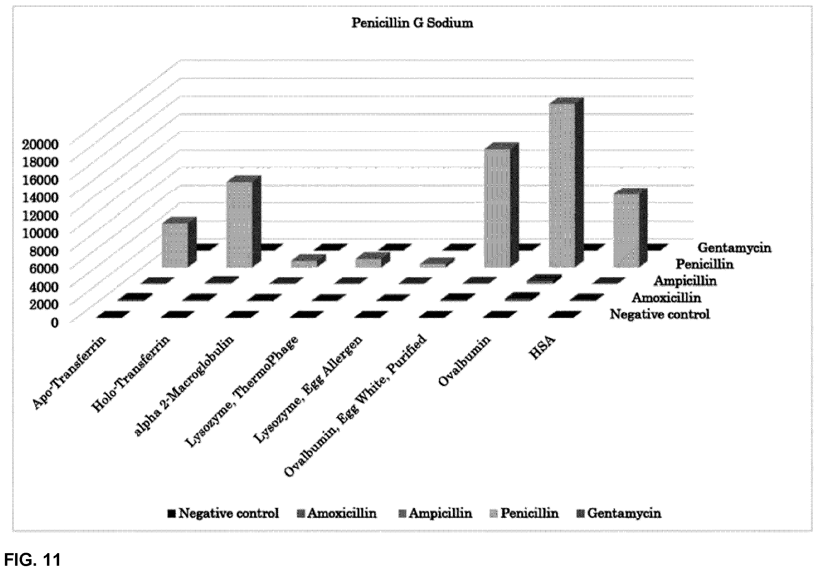

Fig. 11 is a graphical representation of the red intensity values in the table of Fig. 9.

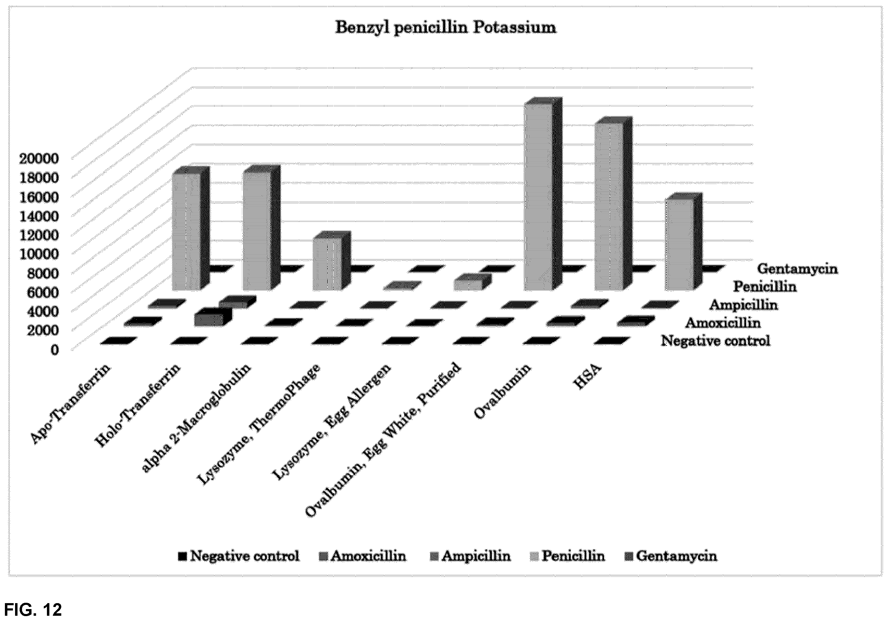

Fig. 12 is a graphical representation of the red intensity values in the table of Fig. 10.

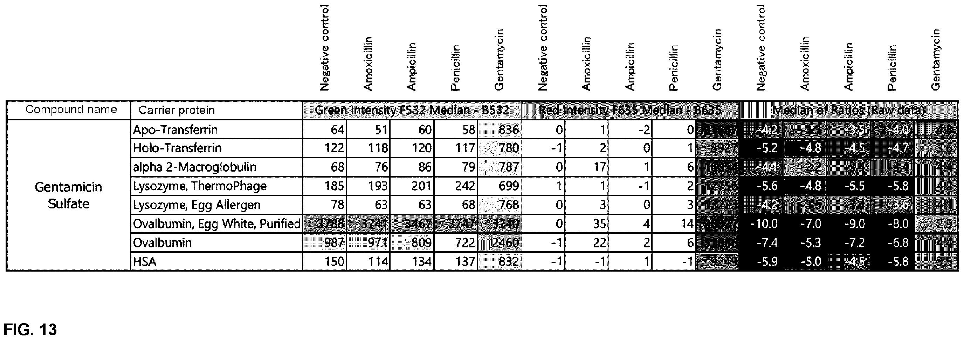

Fig. 13 is a table showing the results of immobilizing a complex of an immobilizing protein and gentamycin sulfate onto a substrate, and detecting each compound on the substrate by using an anti-amoxicillin antibody, an anti-ampicillin antibody, an anti-penicillin antibody, and an anti-gentamycin antibody as well as a fluorescence-labeled secondary antibody in Example 4 described below.

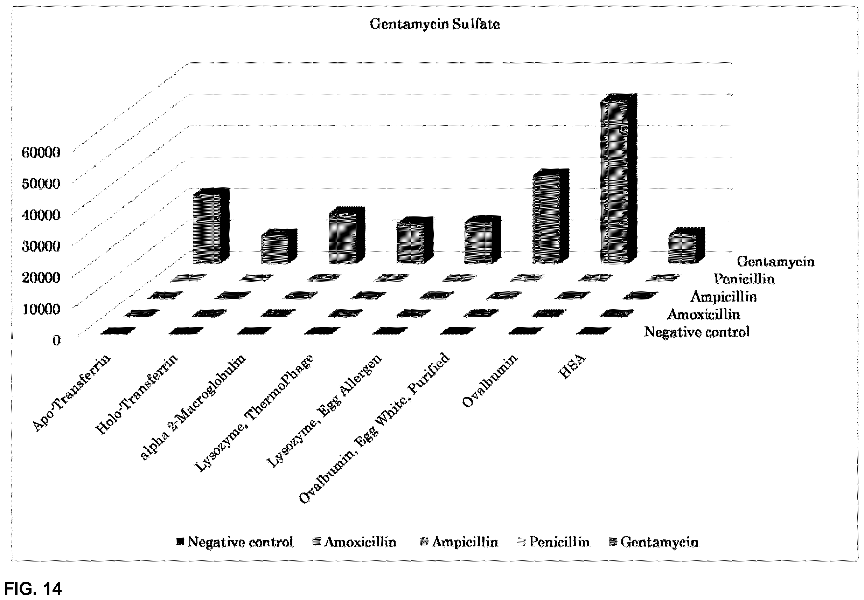

Fig. 14 is a graphical representation of the red intensity values in the table of Fig. 13.

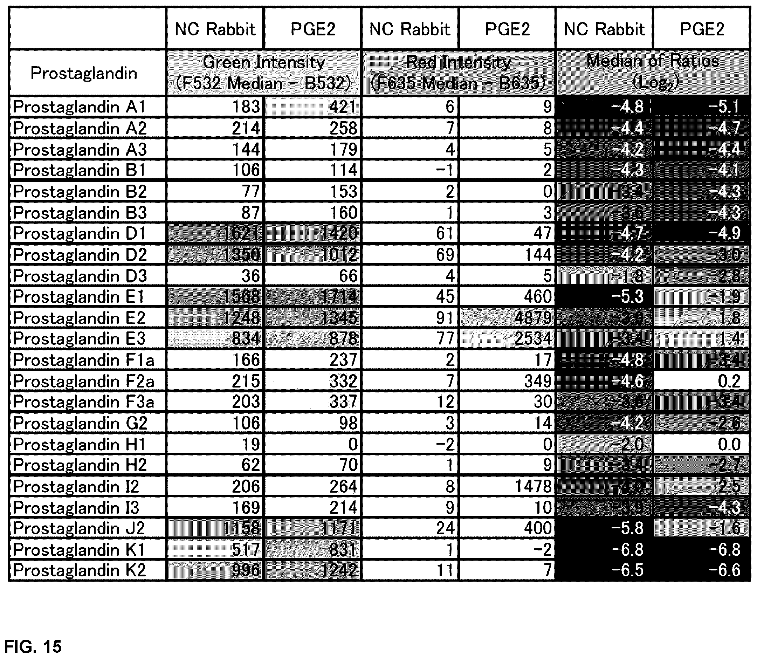

Fig. 15 is a table showing the results of immobilizing complexes of various prostaglandins and HSA onto a substrate, and detecting each prostaglandin on the substrate by using an anti-prostaglandin E2 antibody and a fluorescence-labeled secondary antibody in Example 5 described below.

### DESCRIPTION OF THE PREFERRED EMBODIMENTS

### 1. Method of Immobilizing Compound onto Substrate

### 1-1. Overview

One aspect of the present invention relates to a method of immobilizing a compound with a wide variety of structures and functional groups onto a substrate without requiring modification and while maintaining the structures by a uniform method.

The method of immobilizing a compound onto a substrate of the present invention includes immobilizing the compound as a protein-compound complex onto the substrate via an immobilizing protein for immobilizing the compound onto the substrate.

"Immobilizing a compound onto a substrate via an immobilizing protein" refers to an immobilizing protein being bonded to a substrate, and a compound being bonded to the immobilizing protein, resulting in immobilization of the compound onto the substrate. In this method of immobilizing a compound onto a substrate, a complex of the compound and the immobilizing protein may be prepared first and then the complex may be immobilized onto the substrate, or the immobilizing protein may be bonded to the substrate first and then the compound may be bonded to the immobilizing protein.

That is, in the immobilization method of the present invention, in one embodiment, the immobilization includes
(a) preparing a protein-compound complex by mixing the immobilizing protein and the compound in a solvent, and
(b) immobilizing the protein-compound complex onto the substrate.

Further, in the immobilization method of the present invention, in another embodiment, the immobilization includes
(a') immobilizing the immobilizing protein onto the substrate, and
(b')forming a protein-compound complex by bringing the compound into contact with the immobilizing protein immobilized onto the substrate.

### 1-2. Definitions

In this specification, a "substrate" is not limited as long as capable of immobilizing a protein-compound complex via an immobilizing protein, and being used for detection of a compound immobilized onto a substrate and the like. Examples of such a substrate include inorganic materials such as metals, metal oxides, glass, quartz, silicon, and ceramics, synthetic polymers such as elastomers, plastics, polyester resins, polyethylene resins, polypropylene resins, acrylonitrile butadiene styrene (ABS) resins, nylons, acrylic resins, fluorine resins, polycarbonate resins, polyurethane resins, methylpentene resins, phenol resins, melamine resins, epoxy resins, and vinyl chloride resins, and natural polymers such as chitin, chitosan, and cellulose. A shape of the substrate is not limited, and examples include a flat shape such as a flat plate, a flat film, a film, or a porous film, or a three-dimensional shape such as a cylinder, a stamp, a multiwell plate, a microchannel, or a fine particle. As the substrate, a substrate composed of glass, quartz, or silicon is more preferred, and glass is even more preferred.

"Glass" is a transparent compound with silicate as a main component. The glass that can be used for the substrate of the present invention is not limited as long as capable of immobilizing a compound via an immobilizing protein, and may include chemical components other than silicate. Examples include, but are not limited to, soda lime glass, borosilicate glass, and lime glass. From the standpoint of industrial use, the glass is desirably, but not limited to, a glass plate or a glass slide.

An "immobilizing protein for immobilizing a compound onto a substrate" that can be used in the present invention is not limited as long as capable of immobilizing a compound onto a substrate (preferably glass) defined in this specification, and examples include blood proteins. Such an immobilizing protein is not limited to a biosynthesized protein, and a chemically synthesized protein can also be used. An origin of the immobilizing protein is not limited and is preferably a blood protein derived from a mammal (human, monkey, cow, sheep, rabbit, rat, guinea pig, mouse, or the like) or a bird.

An immobilizing protein in this specification includes variants and modifications or portions thereof, as long as the protein has properties capable of immobilizing a compound onto a substrate. A "variant" refers to, for example, a protein composed of an amino acid sequence in which one or several amino acids are deleted, substituted, or added in an amino acid sequence constituting the immobilizing protein, and has properties capable of forming a complex with a compound and immobilizing the compound onto a substrate. A "modification" refers to a protein obtained by chemically modifying a portion of an amino acid sequence of the protein to add another chemical structure to the amino acid sequence of the immobilizing protein, or a protein obtained by removing a portion of the chemical structure of the immobilizing protein, and has properties capable of forming a complex with a compound and immobilizing the compound onto a substrate.

A "blood protein" in this specification refers to a protein included in blood. Examples of blood proteins that can be used in the present invention include albumin (serum albumin such as human serum albumin (HSA) and bovine serum albumin (BSA), and the like), α-globulin (α1-antitrypsin, haptoglobin, α2-macroblobulin, ceruloplasmin, thyroxine-binding globulin, and the like), transferrin (apo-transferrin, holo-transferrin, and the like), lysozyme, apolipoprotein, and transthyretin. Further, as long as capable of forming a complex with a compound and immobilizing of the compound onto a substrate, portions of these blood proteins are also included.

Further, an "analog of a blood protein" in this specification refers to a protein, or a modification or a portion thereof, that is derived from a source other than blood and has similar properties to a specific blood protein, and is capable of immobilizing a compound onto a substrate. Examples of analogs of a blood protein derived from a source other than blood include ovalbumin, lactalbumin, ovotransferrin, and lactotransferrin.

The blood protein may be obtained by being isolated from serum collected from a living body or being artificially synthesized (including biosynthesis and chemical synthesis). Methods of preparing serum from a living body and isolating and purifying each blood protein from the serum are well known. Further, commercially available blood proteins can also be used. It should be noted that, in a preferred embodiment, the blood protein used in the present invention is a serum protein.

A "compound" in this specification includes low-molecular compounds, high-molecular compounds, organic compounds, inorganic compounds, polysaccharides, polymers, lipids, nucleic acids and analogs thereof, polypeptides, proteins, antibodies, lectins, and compounds constituted by combinations thereof. The compounds immobilized onto a substrate in the present invention are not limited as long as capable of dissolving in a solvent and forming a complex with an immobilizing protein for immobilizing the compound onto the substrate. The compound may be a naturally existing compound or an artificially synthesized compound. Further, the compounds that form protein-compound complexes may be identical to each other across the protein-compound complexes, or may be two or more compounds that differ from each other.

A "low-molecular compound" refers to a compound having a molecular weight of approximately 10000 or less and distinguished from high-molecular compounds, nucleic acids, and polypeptides, and examples include, but are not limited to, amoxicillin, ampicillin, apramycin, bacitracin, cefalexin, ceftazidime, cloxacillin, gentamicin, hygromycin B, neomycin, penicillin G, streptomycin, sulfadimidine, and tetracycline.

A "high-molecular compound" refers to a compound having a molecular weight exceeding approximately 10000 and distinguished from low-molecular compounds, nucleic acids, and polypeptides, and examples include, but are not limited to, starch, cellulose, synthetic fibers, plastics, rubber, asbestos, and phosphonitrilic chloride polymer.

A "polysaccharide" refers to a compound consisting of a plurality of polymerized monosaccharides such as glucose and mannose, and a type of monosaccharide constituting the polysaccharide, a bonding method, a molecular weight, and forms of a main chain and a side chain are not limited. Examples include, but are not limited to, acidic polysaccharides including carrageenan, pectin, gum arabic, xanthan gum, gellan gum, agar, and tragacanth gum; neutral polysaccharides including tamarind seed gum, guar gum, locust bean gum, starch, and pullulan; and basic polysaccharides including chitosan.

An "organic compound" refers to a compound in which carbon is the main element of atomic bonds, and "inorganic compound" refers to a compound in which carbon is not included in atomic bonds. A "polymer" refers to a compound formed by the polymerization of two or more monomers. A "lipid" refers to an organic compound that exists in a living organism and is insoluble in water and soluble in organic solvents. Lipids include, but are not limited to, bioactive substances such as prostaglandins. A "nucleic acid" refers to deoxyribonucleic acid (DNA), ribonucleic acid (RNA), or the like. A "polypeptide" refers to a polymer in which peptide bonds are formed by a large number of amino acids through a polycondensation reaction. The compounds enumerated above are also not particularly limited and are the target compounds immobilized onto a substrate in the immobilization method of the present invention.

### 1-3. (a) Preparing protein-compound complex by mixing immobilizing protein and compound in solvent

Immobilizing a compound via an immobilizing protein onto a substrate of the present invention, in one embodiment, includes the above-described step (a) preparing a protein-compound complex by mixing an immobilizing protein and a compound in a solvent.

The solvents that can be used in the step (a) are not limited as long as the immobilizing protein and the compound dissolve and the protein-compound complex can be formed in the solvent. Suitably, examples include solvents normally used for immobilization or detection in detection techniques for various compounds immobilized onto a substrate, such as compound microarrays, DNA microarrays, and protein microarrays. Such solvents are well known, and commercially available solvents may be used. Examples include, but are not limited to, distilled water, dimethyl sulfoxide (DMSO), methyl acetate, acetone, and mixtures thereof.

Conditions for mixing an immobilizing protein and a compound in the solvent are not limited as long as the protein-compound complex can be formed. The conditions for mixing vary depending on the immobilizing protein and the compound to be used, and can be set as appropriate by a person skilled in the art. Further, concentrations of the immobilizing protein and the compound are also not limited as long as the protein-compound complex can be formed, and can be set as appropriate for each immobilizing protein and target compound to be used. The concentration of the immobilizing protein can be set to within a range from 0.1 mg/ml to 10 mg/ml, and is preferably a concentration within a range from 0.4 mg/ml to 1.0 mg/ml, but is not limited thereto.

The solvent may further include additives such as stabilizers.

Here, a "protein-compound complex" refers to a complex formed by the bonding of a compound and an immobilizing protein for immobilizing a compound onto a substrate in a solvent. The immobilizing protein and the compound may be bonded one to one, or may be bonded at a ratio of one to a plurality, a plurality to one, or a plurality to a plurality.

In one embodiment, a "protein-compound complex" refers to a complex in which a protein and a compound are bonded together without a specific bonding form between one specific atom or one specific structure. In other words, when a plurality of protein-compound complexes are present, the immobilizing proteins and the compounds are bonded between different atoms or structures in each protein-compound complex. Thus, the compounds forming each complex are bonded to the immobilizing proteins in different orientations across the protein-compound complexes, and surfaces thereof are presented to the outside in different orientations across the protein-compound complexes.

Further, in one embodiment, the "protein-compound complex" may be a complex in which the immobilizing protein and the compound are bonded by a non-covalent bond. Further, in one preferred embodiment, the "protein-compound complex" refers to a complex in which the immobilizing protein and the compound are bonded by physical adsorption.

### 1-4. (b) Immobilizing protein-compound complex onto substrate

Immobilizing a compound via an immobilizing protein for immobilizing the compound onto the substrate of the present invention includes, in one embodiment, (b) immobilizing the protein-compound complex onto the substrate after the above-described step (a).

In this specification, "immobilizing a protein-compound complex onto a substrate" refers to immobilizing the complex onto the substrate via a structure of the immobilizing protein in the protein-compound complex. The complex need only be immobilized onto the substrate with sufficient strength to withstand an intended use and, for example, in a case in which the intention is to detect an antibody that binds to a compound, need only be immobilized onto the substrate with sufficient strength for enabling detection of the antibody that binds to the compound after immobilization. In one embodiment, the bonding to the substrate via the structure of the immobilizing protein in the protein-compound complex may be performed by a non-covalent bond. In one preferred embodiment, the bonding to the substrate via the structure of the immobilizing protein in the protein-compound complex is performed by physical adsorption.

A method of immobilizing a protein-compound complex can include bringing a solution including the protein-compound complex into contact with the substrate. As the solution including the protein-compound complex, the solution prepared in the above-described step (a) may be used as is, or a solution obtained by transfer to another solvent may be used.

A method of bringing a solution including a protein-compound complex into contact with the substrate is not limited as long as the protein of the protein-compound complex can be immobilized onto the substrate by bringing the solution into contact with the substrate. Preferably, the method includes a mode of spotting on the substrate by using a known ultratrace dispensing system (Micro Diagnostic) or the like.

The compound in the protein-compound complex immobilized onto the substrate by the immobilization of the above-described step (b) maintains a structure thereof. "The compound maintains a structure thereof" means that the structure of the compound does not have any modifications for immobilization. Further, the compound in the protein-compound complex retains, in whole or in part, the same structure as that prior to formation of the protein-compound complex. For example, by detecting the compound after formation of the protein-compound complex by using an appropriate detection means, it can be confirmed that the necessary structure has been maintained when the compound is detected. One example of a compound in a protein-compound complex maintaining the structure thereof includes, but is not limited to, retaining the structure of an epitope to which an antibody against the compound binds.

In one preferred embodiment, the compound in the protein-compound complex immobilized onto the substrate maintains activity thereof. The compound maintaining activity thereof includes retaining the same necessary activity as that prior to formation of the protein-compound complex when the compound is detected. Preferably the activity is about the same as that prior to formation of the protein-compound complex, but need only be retained to the extent necessary for detection of the compound. One example includes, but is not limited to, maintaining the activity of an enzyme in a case in which the compound is an enzyme and, in this case, the compound (enzyme) immobilized onto the substrate can be detected by utilizing the enzyme activity.

After the solution including the protein-compound complex is brought into contact with the substrate, preferably the substrate is cleaned, as necessary, to remove contaminants and the like that were not immobilized onto the substrate. Thus, the method of immobilizing a compound onto a substrate of the present invention can, in one embodiment, include cleaning after step (b). This can improve a sensitivity and an accuracy of the detection of the compound immobilized onto the substrate. A solution used for cleaning the substrate is not limited as long as capable of removing contaminants without completely dissociating the bond of the protein-compound complex and the substrate, and known cleaning solutions that can be used in the manufacture of microarray chips and the like can be used. Specific examples include, but are not limited to, ethanol and ultrapure water. The cleaning is preferably performed by, but not limited to, immersing the substrate onto which the protein-compound complex is immobilized in a cleaning solution such as ethanol or ultrapure water, and shaking the mixture for about 30 seconds at room temperature. The cleaning is preferably performed once or a plurality of times and, for example, can be performed once by ethanol, and subsequently twice by ultrapure water.

### 1-5. (a') Immobilizing immobilizing protein onto substrate

Immobilizing a compound via an immobilizing protein for immobilizing the compound onto a substrate of the present invention includes, in one embodiment, (a') immobilizing the immobilizing protein onto the substrate.

A method of immobilizing the immobilizing protein onto the substrate can include bringing a solution including the immobilizing protein into contact with the substrate.

A method of bringing the solution including the immobilizing protein into contact with the substrate is not limited as long as the immobilizing protein can be immobilized onto the substrate by bringing the solution into contact with the substrate. Preferably, the method includes a mode of spotting on the substrate by using a known ultratrace dispensing system (Micro Diagnostic) or the like. As a solvent, a solvent applicable in the above-described step (a) can be used.

### 1-6. (b')Forming protein-compound complex by bringing compound into contact with immobilizing protein immobilized onto substrate

Immobilizing a compound via an immobilizing protein onto a substrate of the present invention includes, in one embodiment, (b') forming a protein-compound complex by bringing the compound into contact with the immobilizing protein immobilized onto the substrate after the above-described step (a').

A method of bringing the compound into contact with the immobilizing protein immobilized onto the substrate can include bringing a solution including the compound into contact with the immobilizing protein on the substrate. Preferably, the method includes a mode of spotting on the substrate by using a known ultratrace dispensing system (Micro Diagnostic) or the like. Conditions under which the solution including the compound is brought into contact with the immobilizing protein on the substrate can be made the same as the solvent and mixing conditions of the method of the above-described step (a) and set as appropriate.

Further, the method of immobilizing a compound onto a substrate of the present invention can, in one embodiment, include cleaning after step (a') and/or step (b'). The cleaning can be performed under the same conditions as those of the cleaning after the above-described step (b).

### 1-7. Recovery

Immobilizing a compound via an immobilizing protein onto a substrate of the present invention includes, in one embodiment, recovering the compound from the substrate after the above-described step (b) or (b'). A method of recovering the compound from the substrate can include recovering the compound as a protein-compound complex by separating the protein-compound complex from the substrate, or can include recovering the compound by separating the compound itself from the protein-compound complex. As the method of recovering the compound, known means can be adopted as appropriate, depending on the substrate and the protein used and the type of compound to be recovered.

### 2. Detecting Compound Immobilized onto Substrate

### 2-1. Overview

Another aspect of the present invention relates to a method of detecting a compound immobilized onto a substrate.

The method of detecting a compound immobilized onto a substrate of the present invention includes detecting a protein-compound complex immobilized onto the substrate by the above-described immobilization method.

### 2-2. Detection

As a method of detecting the protein-compound complex immobilized onto the substrate, a known detection means that is preferred depending on the target compound can be adopted as appropriate. Such a detection means is not limited as long as a means capable of detecting the presence of the compound by binding to or reacting with the compound. Examples include, but are not limited to, antibodies, lectins, probes, receptors, antigens, haptens, nucleic acids (DNA, RNA, or PNA), sugar chains, ligands, aptamers, polynucleotides, lipids, enzymes, and enzyme substrates. Further, as methods of detecting the compound immobilized onto the substrate by using these detection methods, known methods can be adopted. The detection method in a preferred embodiment includes, but is not limited to, the use of a labeled antibody.

### 3. Screening Compound Having Affinity for Compound Immobilized onto Substrate

### 3-1. Overview

Another aspect of the present invention relates to a method of screening a compound having affinity for a target compound immobilized onto a substrate.

A method of screening a compound having affinity for a target compound immobilized onto a substrate of the present invention includes
(i) immobilizing the target compound as a protein-compound complex onto a substrate via an immobilizing protein for immobilizing the compound onto the substrate, and
(ii) detecting a compound having affinity for the target compound by bringing a candidate compound into contact with the target compound immobilized onto the substrate.

Step (i) in the screening method of the present invention can be implemented as in steps (a) and (b) or steps (a') and (b') in the above-described immobilization method.

### 3-2. (ii) Detecting compound having affinity for protein-compound complex by bringing compound into contact with protein-compound complex immobilized onto substrate

As the method of screening a compound having affinity for the compound immobilized onto the substrate of the present invention, after step (i), in step (ii), the candidate compound is brought into contact in order to screen a compound having affinity for the compound constituting the protein-compound complex immobilized onto the substrate.

Methods of bringing a candidate compound into contact to screen a compound having affinity for the compound immobilized onto the substrates are well known, and conditions such as a solvent, a temperature, and a time used when bringing the candidate compound into contact can be set as appropriate depending on the combination of the compound constituting the protein-compound complex and the candidate compound.

After candidate compounds are brought into contact, the compounds having affinity for the compound immobilized onto the substrate among the candidate compounds are detected. As a detection method, known methods can be used as in the detection for a protein-compound complex described above. Further, the candidate compounds detected as compounds having affinity can be recovered from the substrate by a known method as appropriate.

### 3-3. Method of screening anti-compound antibody

One specific embodiment of the method of screening a compound having affinity for a target compound immobilized onto a substrate of the present invention is a method of comprehensively detecting and screening anti-compound antibodies present in a sample by using a compound microarray.

The method includes
(i) immobilizing the target compound as a protein-compound complex onto a substrate via an immobilizing protein for immobilizing the compound onto the substrate, and
(ii')detecting an anti-compound antibody having affinity for the protein-compound complex by bringing antibodies into contact with the protein-compound complex immobilized onto the substrate.

Step (i) in the screening method of the present invention can be implemented as in steps (a) and (b) or steps (a') and (b') in the above-described immobilization method.

In this embodiment, compounds constituting a plurality of protein-compound complexes immobilized onto the substrate in step (ii') are preferably mutually the same compounds.

Further, in step (ii'), a plurality of antibodies having complementarity determining regions (CDRs) that differ from each other can be brought into contact with the protein-compound complex as candidate antibodies to detect, among the candidate antibodies, an anti-compound antibody having affinity. A method of bringing the antibodies into contact with the protein-compound complex immobilized onto the substrate and a method of detecting the antibodies having affinity for the target compound can be performed in accordance with known methods of screening antibodies having affinity for the specific compound.

Further, the candidate antibodies detected as anti-compound antibodies having affinity can be recovered from the substrate by a known method as appropriate.

### 3-4. Test method of biological sample

One specific embodiment of the method of screening a compound having affinity for the target compound immobilized onto the substrate of the present invention is a method of comprehensively detecting the presence of a disease-related marker in a biological sample by using a compound microarray onto which an antibody against the disease-related marker is immobilized.

The method includes
(i) immobilizing a compound having affinity for a disease-related marker as a protein-compound complex onto a substrate via an immobilizing protein for immobilizing the compound onto the substrate, and
(ii") detecting the disease-related marker in the biological sample by bringing a compound derived from the biological sample into contact with the compound having affinity for the disease-related marker and immobilized onto the substrate.

Here, in one preferred embodiment, the target compound is an antibody against a disease-related marker.

A "disease-related marker" is a gene whose expression is elevated or suppressed in relation to a contraction, a risk of contraction, or a prognostic course of a specific disease, and is not particularly limited as long as capable of determining the contraction, the risk of contraction, or the prognostic course of a specific disease. Disease-related markers include transcripts and translation products, the detection of which can determine whether or not a person has, or whether or not a person is at risk of having, a specific disease.

A "biological sample" is a sample collected from a living body and used in the test method of a biological sample of this aspect, and tissue, cells, body fluids, or peritoneal lavage fluid, for example, fall under this category. The term "body fluid" here refers to a liquid biological sample collected from a living body. Examples include blood (including serum, plasma, and interstitial fluid), spinal fluid (cerebrospinal fluid), urine, feces, saliva, lymph fluid, digestive fluid, ascites, pleural fluid, perineural fluid, and extracts of various tissues or cells. The preferred biological sample is blood.

As the compound derived from a biological sample, a compound pretreated by purification, concentration, or the like, in accordance with the objective compound in the biological sample, prior to immobilization as a protein-compound complex onto the substrate, may be used. For the purification or concentration of a biological sample, known methods can be adopted as appropriate in accordance with the objective compound. As the compound derived from a biological sample, a sample collected from the living body can also be used as is as long as capable of being immobilized as a protein-compound complex onto the substrate.

In this embodiment, in step (ii"), the bond between the compound having affinity for the disease-related marker and immobilized onto the substrate and the compound (disease-related marker) in the biological sample is detected. This makes it possible to confirm the presence of the disease-related marker in the biological sample, and thus determine the contraction of the disease, the risk thereof, and the prognostic course.

### 3-5. Test method of biological sample 2

One specific embodiment of the method of screening a compound having affinity for the target compound immobilized onto the substrate of the present invention relates to a method of obtaining a profile of a compound included in the biological sample.

The method includes
(i) immobilizing the target compound as a protein-compound complex onto a substrate via an immobilizing protein for immobilizing the compound onto the substrate, and
(ii‴) detecting a compound derived from a biological sample that has affinity for an antigen-candidate compound by bringing the compound derived from the biological sample into contact with the target compound immobilized onto the substrate.

This embodiment can be implemented to obtain a profile of a compound included in the biological sample. The target compound in this embodiment may be a compound having affinity for a desired compound included in the biological sample. Examples of the compound included in the biological sample in this embodiment include, but are not limited to, an antibody, a lectin, and a nucleic acid.

According to this embodiment, it is possible to obtain a profile of a compound included in the biological sample by detecting whether a compound having affinity for the target compound is included in the biological sample.

### 3-6. Test method of biological sample 3

One specific embodiment of the method of screening a compound having affinity for the target compound immobilized onto the substrate of the present invention relates to a method of obtaining a profile of an antibody included in the biological sample.

The method includes
(i) immobilizing an antigen-candidate compound as a protein-compound complex onto a substrate via an immobilizing protein for immobilizing the compound onto the substrate, and
(iiʺʺ) detecting an antibody derived from a biological sample having affinity for the antigen-candidate compound by bringing the antibody derived from the biological sample into contact with the antigen-candidate compound immobilized onto the substrate.

An "antigen-candidate compound" refers to a compound that can be an antigen in a living organism (preferably, a human). The antigen-candidate compounds are preferably antigens for confirming the presence of an antibody in the living body, and examples include, but are not limited to, compounds derived from allergens, viruses or bacteria that produce allergies (drug allergies, food allergies, metal allergies, hay fever, and the like) in the living body.

According to this embodiment, the method can be used for an allergy (for example, a drug allergy) test or the like by detecting the presence of an antibody against the antigen-candidate compound. For example, knowing in advance that an antibody against a specific compound is present in the blood collected from a living body makes it possible to avoid anaphylactic shock from a drug.

### 3-7. Method of sorting and acquiring cell expressing antigen receptor or antibody-producing cell

One specific embodiment of the method of screening a compound having affinity for the target compound immobilized onto the substrate of the present invention relates to a method of sorting and acquiring a cell that expresses an antigen receptor for or a cell that produces an antibody against the target compound.

The method includes
(i) immobilizing an antigen-candidate compound as a protein-compound complex onto a substrate via an immobilizing protein for immobilizing the compound onto the substrate,
(iiʺ‴) bringing a cell expressing an antigen receptor or an antibody derived from an antibody-producing cell into contact with an antigen-candidate compound immobilized onto the substrate, and
(iii) recovering a cell that expresses an antigen receptor having affinity for or an antibody-producing cell that produces the antibody against the antigen-candidate compound.

A method of sorting and acquiring a cell that expresses an antigen receptor for or a cell that produces an antibody against the target compound may, in one embodiment, include detecting a cell expressing an antigen receptor having affinity for or the antibody against the antigen-candidate compound before step (iii).

The cells expressing an antigen receptor or the antibody-producing cells include, for example, B cells, memory B cells, T cells, plasma cells, lymphoblastoid cell lines, and the like. These cells may be cells derived from biological samples, may be human peripheral blood mononuclear cells infected with a virus such as Epstein-Barr (EB) virus, or may even be artificially prepared or genetically manipulated cells.

A method of bringing a cell expressing an antigen receptor or an antibody derived from an antibody-producing cell into contact with the antigen-candidate compound immobilized onto the substrate is particularly not limited, and a solution including the cell expressing the antigen receptor or the antibody-producing cell (for example, culture medium normally used for culturing the antibody-producing cell) may be brought into contact with the substrate onto which the antigen-candidate compound has been immobilized, or a solution including the antibody derived from antibody-producing cell (for example, culture supernatant of the antibody-producing cell) may be brought into contact with the substrate onto which the antigen-candidate compound is immobilized.

A method of recovering a cell expressing an antigen receptor having affinity for the antigen-candidate compound is not particularly limited, and examples include a method of recovering cells accumulated near the antigen-candidate compound on the substrate, and a method of recovering cells expressing antigen receptors having high affinity for the candidate compound by excluding cells expressing antigen receptors having low affinity through a physical sorting method such as a cleaning operation.

According to this embodiment, the affinity between an antigen receptor or an antibody derived from an antibody-producing cell and an antigen-candidate compound on a substrate can be utilized to sort cells that can produce an antibody specific to the antigen-candidate compound.

### 4. Immobilizing Agent for Immobilizing Compound onto Substrate

### 4-1. Overview

Another aspect of the present invention relates to an immobilizing agent including an immobilizing protein for immobilizing a compound onto a substrate. The substrate, the immobilizing protein for immobilizing a compound onto the substrate, and the compound to be immobilized are the substrate, the immobilizing protein, and the compound described or enumerated above. A preferred embodiment of the immobilizing agent according to the present invention is an immobilizing agent composed of the immobilizing protein for immobilizing the compound onto the substrate.

### 5. Kit

### 5-1. Overview

Another aspect of the present invention relates to a kit for immobilizing a compound onto a substrate. The kit includes a substrate and an immobilizing protein for immobilizing a compound onto the substrate. The kit of the present invention, in one embodiment, can further include a solvent for forming a complex of the immobilizing protein and the compound, a means for detecting the compound, a cleaning solution, and an instrument such as a well.

According to the kit of the present invention, it is possible to easily immobilize a compound with a wide variety of structures and functional groups onto a substrate without requiring separate modification and while maintaining the structures of the compound as is.

The present invention will be described more specifically by means of examples below, but the technical scope of the present invention is not limited to these examples.

### Examples

### (Example 1. Immobilizing compounds onto glass slides by using human serum albumin)

Fourteen compounds (amoxicillin, ampicillin, apramycin, bacitracin, cefalexin, ceftazidime, cloxacillin, gentamicin, hygromycin B, neomycin, penicillin G, streptomycin, sulfadimidine, and tetracycline) were each mixed with human serum albumin (HSA) (Sigma-Aldrich) and printed on glass slides. Further, compounds of bovine serum albumin (BSA) covalently bonded to these 14 compounds in advance (Imgen BioSciences) were printed on glass slides as control samples for comparison. The compounds were each dissolved in solution A for protein microarrays only (Fukushima Protein Factory) to a concentration of 0.5 mM. Furthermore, HSA was prepared and mixed with the solutions A including the compounds, bringing the final concentrations to 0.1 micrograms/microliter. It should be noted that the printing of each compound on a glass slide was performed by spotting microdroplets of approximately 2 to 4 nl on the glass slide by using an ultratrace dispensing system (Micro Diagnostic).

The glass slides printed with the compounds (hereinafter referred to as "compound microarrays") were immersed in ethanol and shaken for 30 seconds at room temperature. Subsequently, the compound microarrays were immersed in ultrapure water and shaken for 30 seconds at 26°C. It should be noted that the shaking by ultrapure water was performed twice. For solution replacement, the compound microarrays were immersed in solution A for protein microarrays only (Fukushima Protein Factory, Fukushima, Japan) and shaken for about five minutes at 26°C.

The compound microarrays treated as described above were immersed in Blocking One (Nacalai Tesque, Kyoto, Japan) and shaken for one hour at 26°C. Subsequently, the compound microarrays were immersed in solution A for protein microarrays only and shaken for about five minutes at 26°C.

Using a solution obtained by adding 1/4000 volume of Goat Reference Antibody Mixture I (Fukushima Protein Factory) to a primary antibody diluent for protein microarrays only (Fukushima Protein Factory), a mouse monoclonal anti-BSA antibody (ab79827; Abeam, Cambridge, UK) or one of six mouse monoclonal anti-HSA antibodies (Proteintech Group; Proteintech, NBP1-79079; Novus Biologicals, NB100-73044; Novus Biologicals, NB100-7903; Novus Biologicals, ab10241; Abcam, and 8260-0025; Bio-Rad) (Table 1) was diluted to 1 microgram/milliliter.

**[Table 1]**

| # | Antigen | Host | Clone | Supplier | Product # | Lot # |
|---|---|---|---|---|---|---|
| NC | | | | | | |
| 1 | BSA | mouse | 1C12 | abeam | ab79827 | GR275182-2 |
| 2 | HSA | mouse | 4A1C11 | Proteintech | Proteintech Group | 10000908 |
| 3 | HSA | mouse | HSA20 | Novus | NBP1-79049 | 11/06-T24-20 |
| 4 | HSA | mouse | 6B11 | Novus | NB100-73044 | 15105-T24-B11 |
| 5 | HSA | mouse | 15C7 | Novus | NB110-7903 | 14/09-T24-C7 |
| 6 | HSA | mouse | 15C7 | abcam | ab10241 | GR3197685-1 |
| 7 | HSA | mouse | AL-01 | Bio-Rad | 8260-0025 | 170720 |

Two milliliters of each solution with the diluted primary antibody were added to a P-DE1 cassette for protein microarrays (Fukushima Protein Factory). The compound microarrays were immersed therein, the lid of the cassette was placed thereon, the cassette was sealed with a special clip, and reactions were permitted with shaking for about 17 hours at 37°C.

After the reaction with the primary antibody, the compound microarrays were immersed in solution A for protein microarrays only and shaken for one minute at 26°C. It should be noted that this process was repeated three times.

1/500 volume of Cy3-labeled anti-goat IgG antibodies (Jackson) and Alexa Fluor 647-labeled anti-mouse IgG antibodies (Jackson) were added to a secondary antibody diluent for protein microarrays only, and 2 milliliters of the solution were added to a P-DE1 cassette for protein microarrays. The compound microarrays were immersed therein, the lid of the cassette was placed thereon, the cassette was sealed with a special clip, and reactions were permitted with shaking for about one hour at 26°C.

After the reaction with the secondary antibodies, the compound microarrays were immersed in solution A for protein microarrays only and shaken for one minute at 26°C. It should be noted that this process was repeated three times.

The compound microarrays were immersed in solution B for protein microarrays only (Fukushima Protein Factory) and shaken for three minutes at 26°C. It should be noted that this process was repeated three times.

The compound microarrays were immersed in a final cleaning solution for protein microarrays only (Fukushima Protein Factory) and shaken for three minutes at 26°C. It should be noted that this process was repeated four times.

The compound microarrays were dried, centrifuged at 1500 rpm for one minute, and scanned for fluorescence intensity using a GenePix 4000B (Molecular Devices, CA, USA). The results are shown in Fig. 1. In Fig. 1, Green Intensity indicates the fluorescence intensity from the Cy3-labeled anti-goat IgG antibodies, Red Intensity indicates the fluorescence intensity from the Alexa Fluor 647-labeled anti-mouse IgG antibodies, and Median of Ratios indicates the relative value of Red Intensity to Green Intensity (Log₂ value) (the same hereafter).

In the case of the reaction with the anti-BSA antibody, signals were detected only in spots printed with a compound in which BSA was covalently bonded in advance, regardless of the type of compound (column 1 of Red Intensity in Fig. 1). This indicates that BSA can be immobilized on a glass slide and, as long as BSA and the compound can be covalently bonded, that BSA and the compound covalently bonded can be immobilized onto the glass slide.

Further, in the case of the reactions with the six different anti-HSA antibodies, signals were detected only in spots where a compound was mixed with HSA in all anti-HSA antibody test sections (columns 2 to 7 of Red Intensity in Fig. 1). This indicates that at least HSA can be immobilized onto glass slides.

### (Example 2. Verification of immobilization of compounds)

Fourteen compounds (amoxicillin, ampicillin, apramycin, bacitracin, cefalexin, ceftazidime, cloxacillin, gentamicin, hygromycin B, neomycin, penicillin G, streptomycin, sulfadimidine, and tetracycline) were each mixed with human serum albumin (HSA) (Sigma-Aldrich) and printed on glass slides. Further, compounds of bovine serum albumin (BSA) covalently bonded to these 14 compounds in advance (Imgen BioSciences) were printed on glass slides as control samples for comparison. The compounds mixed with HSA were each dissolved in solution A for protein microarrays only (Fukushima Protein Factory) to a concentration of 0.5 mM. Furthermore, HSA was prepared and mixed with the solutions A including the compounds, bringing the final concentrations to 0.1 micrograms/microliter. It should be noted that the printing of each compound on a glass slide was performed by spotting microdroplets of approximately 2 to 4 nl on the glass slide by using an ultratrace dispensing system (Micro Diagnostic).

The compound microarrays printed with the compounds were immersed in ethanol and shaken for 30 seconds at room temperature. Subsequently, the compound microarrays were immersed in ultrapure water and shaken for 30 seconds at 26°C. It should be noted that the shaking by ultrapure water was performed twice. For solution replacement, the compound microarrays were immersed in solution A for protein microarrays only (Fukushima Protein Factory, Fukushima, Japan) and shaken for about five minutes at 26°C.

The compound microarrays treated as described above were immersed in Blocking One (Nacalai Tesque, Kyoto, Japan) and shaken for one hour at 26°C. Subsequently, the compound microarrays were immersed in solution A for protein microarrays only and shaken for about five minutes at 26°C.

Using a solution obtained by adding 1/4000 volume of Goat Reference Antibody Mixture I (Fukushima Protein Factory) to the primary antibody diluent for protein microarrays only (Fukushima Protein Factory), one of eight mouse monoclonal anti-compound antibodies (Table 2) was diluted to 1 microgram/milliliter.

**[Table 2]**

| Target | Clone | Manufacturer (Cat No., Lot No.) |
|---|---|---|
| 1 Negative control | | - |
| 2 Amoxicillin | 1.BB.832 | GeneTex (GTX15054, 821300650) |
| 3 Ampicillin | 164-12-2 | Santa Cruz Biotechnology, Inc. (sc-57622, G2815) |
| 4 Penicillin | P2B9 | Abeam (ab116641, GR3213730-1) |
| 5 Penicillin | Pen-9 | Abeam (ab69234, GR3214096-1) |
| 6 Gentamycin | 1.BB.894 | GeneTex (GTX15062, 14219) |
| 7 Streptomycin | 1.BB.944 | GeneTex (GTX15071, 19431) |
| 8 Dihydrostreptomycin | 1.BB.891 | GeneTex (GTX15059, 22594) |
| 9 Streptomycin/Dihydrostreptomycin | CH-2013 | GeneTex (GTX39076. 821801171) |

Two milliliters of the solution with the diluted primary antibody were added to a P-DE1 cassette for protein microarrays (Fukushima Protein Factory). The compound microarrays were immersed therein, the lid of the cassette was placed thereon, the cassette was sealed with a special clip, and reactions were permitted with shaking for about 17 hours at 37°C.

After the reaction with the primary antibody, the compound microarrays were immersed in solution A for protein microarrays only and shaken for one minute at 26°C. It should be noted that this process was repeated three times.

1/500 volume of Cy3-labeled anti-goat IgG (Jackson) and Alexa Fluor 647-labeled anti-mouse IgG antibody (Jackson) were added to a secondary antibody diluent for protein microarrays only, and 2 milliliters of the solution were added to a P-DE1 cassette for protein microarrays. The compound microarrays were immersed therein, the lid of the cassette was placed thereon, the cassette was sealed with a special clip, and reactions were permitted with shaking for about one hour at 26°C.

After the reaction with the secondary antibodies, the compound microarrays were immersed in solution A for protein microarrays only and shaken for one minute at 26°C. It should be noted that this process was repeated three times.

The compound microarrays were immersed in solution B for protein microarrays only (Fukushima Protein Factory) and shaken for three minutes at 26°C. It should be noted that this process was repeated three times.

The compound microarrays were immersed in a final cleaning solution for protein microarrays only (Fukushima Protein Factory) and shaken for three minutes at 26°C. It should be noted that this process was repeated four times.

The compound microarrays were dried, centrifuged at 1500 rpm for one minute, and scanned for fluorescence intensity using a GenePix 4000B (Molecular Devices, CA, USA). The results of the fluorescence intensity measurements are shown in Fig. 2, and images under an optical microscope of the presence or absence of fluorescence in each microdroplet spotted on the substrate are shown in Fig. 3.

The compounds having strong signals (red intensity of 2000 or greater; the same below) and detected with anti-amoxicillin antibodies were in spots printed with amoxicillin and ampicillin covalently bonded to BSA in advance, and amoxicillin and ampicillin mixed with HSA (Red Intensity in Fig. 2). The compound having a strong signal and detected with anti-ampicillin antibodies was ampicillin mixed with HSA only (Red Intensity in Fig. 2). The compounds having strong signals and detected with anti-penicillin antibodies [Pen-9] were amoxicillin covalently bonded to BSA in advance, and amoxicillin, ampicillin, and penicillin G mixed with HSA (Red Intensity in Fig. 2). The compounds having strong signals and detected with anti-gentamycin antibodies were gentamycin covalently bonded to BSA in advance, and gentamycin mixed with HSA (Red Intensity in Fig. 2). It should be noted that, although a strong signal was not detected with anti-penicillin [P2B9] antibodies, a weak signal was detected with penicillin mixed with HSA (Red Intensity in Fig. 2).

Further, although a strong signal was not detected with anti-streptomycin antibodies, anti-dihydrosterptomycin [1.BB.891], and streptomycin/dihydrostreptomycin [CH-2013], a weak signal was detected with streptomycin mixed with HSA (Red Intensity in Fig. 2). Only weak signals being detected with these antibodies is conceivably a characteristic of these antibodies (that is, a somewhat weak bonding strength). These results indicate that compounds mixed with HSA can be immobilized onto glass slides.

Furthermore, of the seven compounds excluding gentamycin, stronger signals were detected for compounds immobilized by mixture with HSA than for compounds covalently bonded to BSA in advance (Figs. 2 and 3). This result indicates that the present invention, which achieves immobilization by mixture with HSA, is superior to the related art. Further, conceivably, when HSA forms a complex with a compound, HSA and the compound form a complex with each other in various orientations, which exposes the epitope of the antibody used in this test, obtaining high sensitivity results. On the other hand, this implies that the compounds covalently bonded to BSA in advance could not expose the epitope to the antibody in some cases.

### (Example 3. Dependence on compound concentration)

It was verified whether the detection sensitivity of compounds mixed with HSA and immobilized onto glass slides increases depending on the concentration (or amount) of HSA.

Compound microarrays were prepared in the same manner as that described above, with concentrations of HSA mixed with amoxicillin, amoxicillin sodium, penicillin G sodium, and gentamycin sulfate from 0.5 mM to 0.0005 mM. Subsequently, the signal of each spot was detected by using anti-amoxicillin antibodies, anti-penicillin antibodies, or anti-gentamycin antibodies by the same method as that described above. The results showed that, when amoxicillin, penicillin G sodium, and gentamycin sulfate were each mixed with HSA and immobilized onto glass slides, the signal intensity increased in an HSA concentration-dependence. Further, even in the case of amoxicillin sodium and HSA being mixed and immobilized onto a glass slide, although the strongest signal was detected in the case of a compound mixed with 0.05 mM of HSA, the compound tended to be concentration-dependent (Fig. 4).

### (Example 4. Immobilization of compounds by mixture with immobilizing protein)

The possibility of compound immobilization in a case in which the compounds were mixed with proteins included in serum other than HSA and BSA was verified.

As the blood protein or analogs thereof, apo-transferrin, holo-transferrin, alpha 2-macroglobulin, lysozyme (ThermoPhage), lysozyme (egg allergen), chicken, ovalbumin (egg white, purified), and ovalbumin were used. Each blood protein was mixed with each compound (amoxicillin, amoxicillin sodium, penicillin G sodium, benzyl penicillin potassium, or gentamycin sulfate) in solution A to form a protein-compound complex. Each blood protein was dissolved to a final concentration of 0.1 micrograms/microliter and each compound was prepared to a final concentration of 0.5 mM. Compound microarrays were prepared in the same way as that described above by using these solutions including the protein-compound complexes. Subsequently, the signal of each spot was detected by using anti-amoxicillin antibodies, anti-penicillin antibodies, or anti-gentamycin antibodies by the same method as that described above. Further, as a negative control, the fluorescent signal of the sample was measured without an antibody reaction.

The compounds of amoxicillin or amoxicillin sodium mixed with each immobilizing protein or HSA and immobilized onto glass slides resulted in strong signal detection only when reacted with anti-amoxicillin antibodies (Figs. 5 to 8).

The compounds of penicillin G sodium or benzyl penicillin potassium mixed with each immobilizing protein or HSA and immobilized onto glass slides resulted in strong signal detection only when reacted with anti-penicillin antibodies (Figs. 9 to 12).

The compound of gentamycin sulfate mixed with each immobilizing protein or HSA and immobilized onto glass slides resulted in strong signal detection only when reacted with anti-gentamycin sulfate antibodies (Figs. 13 and 14).

### 4-1. Effect of mixture with immobilizing protein on amoxicillin detection sensitivity

When amoxicillin and alpha 2-macroglobulin were mixed and immobilized onto a glass slide, the signal intensity exhibited was approximately 12.5 times higher than when amoxicillin and HSA were mixed and immobilized onto a glass slide, and when amoxicillin and ovalbumin were mixed and immobilized onto a glass slide, the signal intensity detected was approximately 8.2 times higher than when amoxicillin and HSA were mixed and immobilized onto a glass slide. Further, when amoxicillin and lysozyme (ThermoPhage) or lysozyme (egg allergen) were mixed and immobilized onto a glass slide, the signal intensities detected were approximately 2.8 times and approximately 2.4 times higher than when amoxicillin and HSA were mixed and immobilized, respectively.

### 4-2. Effect of mixture with immobilizing protein on amoxicillin sodium detection sensitivity

When amoxicillin sodium and alpha 2-macroglobulin were mixed and immobilized onto a glass slide, the signal intensity detected was approximately 9.9 times higher than when amoxicillin sodium and HSA were mixed and immobilized onto a glass slide, and when amoxicillin sodium and ovalbumin (egg white) were mixed and immobilized onto a glass slide, the signal intensity detected was approximately 4.2 times higher than when amoxicillin sodium and HSA were mixed and immobilized. Further, when amoxicillin sodium and lysozyme (ThermoPhage) or lysozyme (egg allergen) were mixed and immobilized onto a glass slide, the signal intensities detected were approximately 2.8 times and approximately 2.3 times higher than when amoxicillin sodium and HSA were mixed and immobilized, respectively.

### 4-3. Effect of mixture with immobilizing protein on penicillin G sodium detection sensitivity

When penicillin G sodium and ovalbumin or ovalbumin (egg white) were mixed and immobilized onto a glass slide, the signal intensities detected were approximately 2.3 times and approximately 1.6 times higher than when penicillin G sodium and HSA were mixed and immobilized onto a glass slide, respectively, and when penicillin G sodium and holo-transferrin were mixed and immobilized onto a glass slide, the signal intensity detected was approximately 1.2 times higher than when penicillin G sodium and HSA were mixed and immobilized onto a glass slide.

### 4-4. Effect of mixture with immobilizing protein on benzyl penicillin potassium detection sensitivity

When benzyl penicillin potassium and ovalbumin (egg white) or ovalbumin were mixed and immobilized onto a glass slide, the signal intensities detected were approximately 2.1 times and approximately 1.8 times higher than when benzyl penicillin potassium and HSA were mixed and immobilized onto a glass slide, respectively, and when benzyl penicillin potassium and apo-transferrin or holo-transferrin were mixed and immobilized onto a glass slide, the signal intensities detected were both approximately 1.3 times higher than when benzyl penicillin potassium and HSA were mixed and immobilized onto a glass slide.

### 4-5. Effect of mixture with immobilizing protein on gentamycin sulfate detection sensitivity

When gentamycin sulfate and ovalbumin (egg white) or ovalbumin were mixed and immobilized to a glass slide, the signal intensities detected were approximately 5.6 times and approximately 3.0 times higher than when gentamycin sulfate and HSA were mixed and immobilized onto a glass slide, respectively, and when gentamycin sulfate and apo-transferrin were mixed and immobilized onto a glass slide, the signal intensity detected was approximately 2.4 times higher than when gentamycin sulfate and HSA were mixed and immobilized onto a glass slide. Further, when gentamycin sulfate and alpha 2-macroglobulin were mixed and immobilized onto a glass slide, the signal intensity detected were approximately 1.7 times higher than when gentamycin sulfate and HSA were mixed and immobilized onto a glass slide. Furthermore, when gentamycin sulfate and lysozyme (ThermoPhage) or lysozyme (egg allergen) were mixed and immobilized onto a glass slide, the signal intensities detected were both approximately 1.4 times higher than when gentamycin sulfate and HSA were mixed and immobilized onto a glass slide.

### (Example 5. Immobilization of bioactive lipids)

The immobilization of prostaglandins, which are bioactive lipids, was verified. In this example, as the prostaglandins, prostaglandins A1, A2, A3, B1, B2, B3, D1, D2, D3, E1, E2, E3, F1a, F2a, F3a, G2, H1, H2, I2, I3, J2, K1, and K2 were used. The prostaglandins were mixed with HSA in the solvents and at the final concentrations shown in the table below to form prostaglandin and HSA complexes. It should be noted that HSA was added to achieve a final concentration of 0.1 milligram/milliliter.

**[Table 3]**

| **Prostaglandin** | Solvent | Concentration (mM) | Carrier |
|---|---|---|---|
| **Prostaglandin A1** | **DMSO** | **0.5** | **HSA** |
| **Prostaglandin A2** | Methyl acetate solution | **0.5** | **HSA** |
| **Prostaglandin A3** | Methyl acetate solution | **0.015** | **HSA** |
| **Prostaglandin B1** | **DMSO** | **0.5** | **HSA** |
| **Prostaglandin B2** | Methyl acetate solution | **0.5** | **HSA** |
| **Prostaglandin B3** | Methyl acetate solution | **0.015** | **HSA** |
| **Prostaglandin D1** | **DMSO** | **0.5** | **HSA** |
| **Prostaglandin D2** | **DMSO** | **0.5** | **HSA** |
| **Prostaglandin D3** | Methyl acetate solution | **0.014** | **HSA** |
| **Prostaglandin E1** | **DMSO** | **0.5** | **HSA** |
| **Prostaglandin E2** | **DMSO** | **0.5** | **HSA** |
| **Prostaglandin E3** | Methyl acetate solution | **0.143** | **HSA** |
| **Prostaglandin F1a** | **DMSO** | **0.5** | **HSA** |
| **Prostaglandin F2a** | **DMSO** | **0.5** | **HSA** |
| **Prostaglandin F3a** | Methyl acetate solution | **0.142** | **HSA** |
| **Prostaglandin G2** | Acetone solution | **0.014** | **HSA** |
| **Prostaglandin H1** | Acetone solution | **0.014** | **HSA** |
| **Prostaglandin H2** | Acetone solution | **0.014** | **HSA** |
| **Prostaglandin I2** | **DMSO** | **0.5** | **HSA** |
| **Prostaglandin 13** | **DMSO** | **0.05** | **HSA** |
| **Prostaglandin J2** | Methyl acetate solution | **0.149** | **HSA** |
| **Prostaglandin K1** | **DMSO** | **0.5** | **HSA** |
| **Prostaglandin K2** | Methyl acetate solution | **0.143** | **HSA** |

Solutions including each prepared prostaglandin and HSA were spotted and printed on glass slides as microdroplets of approximately 2 to 4 nl by using an ultratrace dispensing system (Micro Diagnostic).

The compound microarrays printed with the prostaglandins were immersed in ethanol and shaken for 30 seconds at room temperature. Subsequently, the compound microarrays were immersed in ultrapure water and shaken for 30 seconds at 26°C. The shaking by ultrapure water was performed twice. For solution replacement, the compound microarrays were immersed in solution A for protein microarrays only (Fukushima Protein Factory, Fukushima, Japan) and shaken for about five minutes at 26°C.

The compound microarrays treated as described above were immersed in Blocking One (Nacalai Tesque, Kyoto, Japan) and shaken for one hour at 26°C. Subsequently, the compound microarrays were immersed in solution A for protein microarrays only and shaken for about five minutes at 26°C.

Using a solution obtained by adding 1/4000 volume of Goat Reference Antibody Mixture I (Fukushima Protein Factory) to the primary antibody diluent for protein microarrays only (Fukushima Protein Factory), an anti-prostaglandin E2 antibody (Ab00484-23.0, Absolute Antibody) was diluted to 1 microgram/milliliter.

Two milliliters of the solution with the diluted primary antibody were added to a P-DE1 cassette for protein microarrays (Fukushima Protein Factory). The compound microarrays were immersed therein, the lid of the cassette was placed thereon, the cassette was sealed with a special clip, and reactions were permitted with shaking for about 17 hours at 37°C. After the reaction with the primary antibody, the compound microarrays were immersed in solution A for protein microarrays only and shaken for one minute at 26°C. It should be noted that this process was repeated three times. Further, as the control, a solution obtained by adding only 1/4000 volume of Goat Reference Antibody Mixture I (Fukushima Protein Factory) to the primary antibody diluent for protein microarrays only (Fukushima Protein Factory) was prepared and used as a control solution. Using the control solution, a process of using a primary antibody diluent including anti-prostaglandin E2 antibodies was performed similarly to the above-described process.

1/500 volume of Cy3-labeled anti-goat IgG (Jackson, product number: 805-165-180) and Alexa Fluor 647-labeled anti-rabbit IgG antibodies (Jackson, product number: 711-605-152) were added to a secondary antibody diluent for protein microarrays only, and 2 milliliters of the solution were added to a P-DE1 cassette for protein microarrays. The compound microarrays were immersed therein, the lid of the cassette was placed thereon, the cassette was sealed with a special clip, and reactions were permitted with shaking for about one hour at 26°C.

After the reaction with the secondary antibodies, the compound microarrays were immersed in solution A for protein microarrays only and shaken for one minute at 26°C. It should be noted that this process was repeated three times. The compound microarrays were immersed in solution B for protein microarrays only (Fukushima Protein Factory) and shaken for three minutes at 26°C. It should be noted that this process was repeated three times. The compound microarrays were immersed in a final cleaning solution for protein microarrays only (Fukushima Protein Factory) and shaken for three minutes at 26°C. It should be noted that this process was repeated four times. The compound microarrays were dried, centrifuged at 1500 rpm for one minute, and scanned for fluorescence intensity using a GenePix 4000B (Molecular Devices, CA, USA). The results of fluorescence intensity measurements are shown in Fig. 15.

As the results, the anti-prostaglandin E2 antibody reacted strongly with prostaglandins E2, E3 and I2. Although the prostaglandins have portions similar in structure and thus cross-reactions are expected, these results indicate that lipids such as prostaglandins can also be immobilized onto glass slides by the present invention.

Although examples according to the present invention and modifications based thereon have been described, the present invention is not necessarily limited thereto, and one skilled in the art may find various alternative examples and modifications without departing from the gist of the present invention or the scope of the appended claims.

## Claims

1. A compound immobilizing method of immobilizing a compound onto a substrate, the compound immobilizing method comprising:
immobilizing the compound as a protein-compound complex onto the substrate via an immobilizing protein for immobilizing the compound onto the substrate.

2. The compound immobilizing method according to claim 1, wherein
the substrate is glass.

3. The compound immobilizing method according to claim 1 or 2, wherein
the immobilizing protein is a blood protein or an analog thereof.

4. The compound immobilizing method according to any one of claims 1 to 3, wherein
the immobilization includes
(a) preparing a protein-compound complex by mixing the immobilizing protein and the compound in a solvent, and
(b) immobilizing the protein-compound complex onto the substrate.

5. The compound immobilizing method according to any one of claims 1 to 3, wherein
the immobilization includes
(a') immobilizing the immobilizing protein onto the substrate, and
(b')forming a protein-compound complex by bringing the compound into contact with the immobilizing protein immobilized onto the substrate.

6. The compound immobilizing method according to any one of claims 1 to 5, wherein
the protein-compound complex is formed by a non-covalent bond between the immobilizing protein and the compound.

7. The compound immobilizing method according to any one of claims 1 to 6, wherein
a plurality of the protein-compound complexes are immobilized onto the substrate, and the immobilizing protein and the compound are bonded to each other in different orientations across the plurality of protein-compound complexes.

8. The compound immobilizing method according to any one of claims 1 to 7, wherein
the compound forming the protein-compound complex maintains activity of the compound before formation of the protein-compound complex.

9. The compound immobilizing method according to any one of claims 1 to 8, wherein
the compound is a compound group including a plurality of compounds that differ from each other, and
the immobilization includes immobilizing the compound group onto the substrate via the immobilizing protein.

10. The compound immobilizing method according to claim 4, further comprising:
(c) cleaning the substrate after step (b).

11. The compound immobilizing method according to claim 5, further comprising:
(c') cleaning the substrate after step (a') or/and step (b').

12. A compound detecting method of detecting a compound immobilized onto a substrate, the compound detecting method comprising:
detecting the compound immobilized onto the substrate by the compound immobilizing method described in any one of claims 1 to 11.

13. The compound detecting method according to claim 12, further comprising:
recovering the protein-compound complex or the compound detected by the detection.

14. A compound screening method of screening a compound having affinity for a target compound, the compound screening method comprising:
(i) immobilizing the target compound as a protein-compound complex onto a substrate via an immobilizing protein for immobilizing the compound onto the substrate, and
(ii) detecting the compound having affinity for the target compound immobilized onto the substrate by bringing a candidate compound into contact with the target compound.

15. The compound screening method according to claim 14, wherein
the target compound is a compound having affinity for a disease-related marker, and the candidate compound is a compound derived from a biological sample, and
step (ii) includes detecting the disease-related marker in the biological sample by bringing the compound derived from the biological sample into contact with the compound having affinity for the disease-related marker and immobilized onto the substrate.

16. The compound screening method according to claim 14, wherein
the candidate compound is a compound derived from a biological sample, and
a profile of a compound included in the biological sample with respect to the target compound is obtained.

17. The compound screening method according to claim 16, wherein
the target compound is an antigen-candidate compound,
the candidate compound is an antibody derived from a biological sample, and
a profile of an antibody included in the biological sample with respect to the antigen-candidate compound is obtained.

18. A compound immobilizing agent comprising:
an immobilizing protein for immobilizing a compound onto a substrate.

19. A screening substrate used in the compound detecting method described in claim 12 or 13 or the compound screening method described in claim 14 or 15, wherein
the immobilizing protein is bonded on a surface thereof.

20. The screening substrate according to claim 19, wherein
the immobilizing protein forms a complex with a compound.

21. An immobilizing kit for immobilizing a compound onto a substrate, the immobilizing kit comprising:
a substrate and an immobilizing protein.
